# EUROPEAN PATENT SPECIFICATION

(11) **EP 1 405 597 B1**
(45) Date of publication and mention of the grant of the patent: **08.10.2008**
(21) Application number: 04000220.6
(22) Date of filing: 11.06.2001
(51) Int. Cl.: A61B 5/15

(54) **Safety vacuum syringe for blood sampling conformed to ergonomics**
Ergonomisch gestaltete Sicherheitsvakuumspritze für Blutproben
Seringue de sécurité à forme ergonomique pour prise de sang

(43) Date of publication of application: 07.04.2004
(62) Divisional of application: 01113514.2
(73) Proprietor: Chen, Long-Hsiung, Taipei (TW)
(72) Inventor: Chen, Long-Hsiung, Taipei (TW)
(74) Representative: Casalonga, Axel

(56) References cited:
- WO-A-89/05118
- JP-A- 8 308 818
- JP-A- 10 033 509
- US-A- 3 326 206

## Description

The present invention relates to a safety vacuum syringe for blood sampling conformed to ergonomics, and more particularly to the syringe that not only conforms to ergonomics in use but may receive the conventional needle head and the vacuum tube so as to lower production cost.

Many viruses and diseases or bodily functions can be examined by a blood test, which makes the blood test to be a regular and frequent job. In preventing a patient from infecting lethal virus or germ, the selection of blood sampling tool will be very important during a blood sampling process. Therefore, in order to make sampling more safer, blood sampling must have some degree of improvement.

The needle head of a conventional blood sampling syringe, as revealed in US patent 5,423,758, is disposed at the central axis of the front end of the syringe. During a blood sampling process, the needle of a syringe is stuck into a patient's vein to draw blood from the vein. But the angle between the syringe and the skin surface of a human body is large when blood sampling is processed because the needle head is at the center of syringe. The needle head is difficult to be inserted into the vein to draw blood. This is especially true for the patients whose blood vessels are thinner, because it is easier for the needle to pass through the blood vessel. Thus, blood sampling can not be done smoothly.

The document JP 08 308818 discloses a blood sampling syringe having a hub, a needle main body, a blood-collecting needle holder having a front wall part which closes its front end part. A notched part is formed in the front wall part, the hub being detachably inserted into the notched part diametrally outward.

One objective of the present invention is to provide a safety vacuum syringe for blood sampling conformed to ergonomics, whereby a needle head is eccentrically disposed at the front end of a syringe to lower the blood sampling angle during the blood sampling process allowing blood sampling to be done smoothly.

Another objective of the present invention is to provide a safety vacuum syringe for blood sampling, wherein an inner needle tip of the needle head has a step shape comprising two right angles so that it can be positioned at the center of the needle head to fit the use traditional vacuum blood collecting cup.

Still another objective of the present invention is to provide a conventional needle head that can be used according to a users' need.

The invention provides a vacuum syringe as defined in claim 1.

The present invention can be better understood by detailed description of the following drawings, in which:
FIG. 1 is a longitudinal sectional view of an example before a needle holder is mounted on a syringe.
FIG. 2 is a longitudinal sectional view of an example after a needle holder is mounted on a syringe.
FIG. 3 is a side view of an example showing a guide groove;
FIG. 4 is a longitudinal sectional view of an example, showing the structure inside;
FIG. 4A is a sectional view of an example when viewed along the section line A--A of FIG 4;
FIG. 5 is an explosive view of a reduced lining tube;
FIG. 6 is a longitudinal sectional view of an example during use;
FIG. 7 is a longitudinal sectional view of an example, showing a condition withdrawing a reduced lining tube back;
FIG. 8 is a longitudinal sectional view of an example, showing a needle head being directly inserted into an eccentric reduced portion;
FIG. 9 is a longitudinal sectional view of a preferred embodiment of the present invention, showing a press plate being separated from a syringe;
FIG. 10 is a longitudinal sectional view of a preferred embodiment of the present invention, showing a press plate being wedged into a syringe;
FIG. 11 is an explosive view of the front part of a preferred embodiment of the present invention;
FIG. 12 is a perspective view of the front part of a preferred embodiment of the present invention;
FIG. 13 is a longitudinal sectional view of a preferred embodiment of the present invention during use;

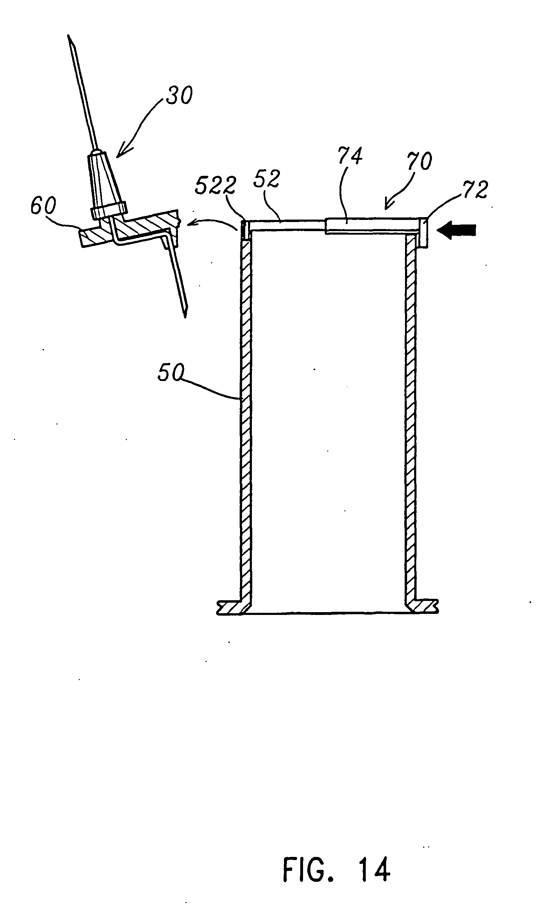

FIG. 14 is a schematic longitudinal sectional view of a preferred embodiment of the present invention, showing a needle seat being separated from a syringe; and

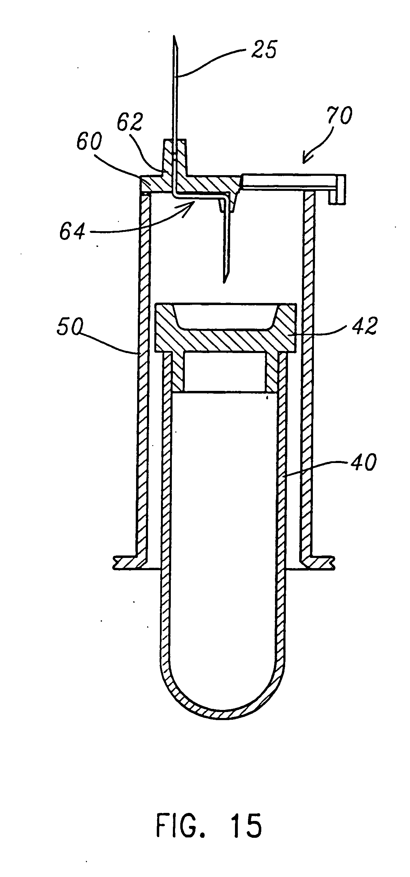

FIG. 15 is a schematic longitudinal sectional view of a preferred embodiment of the present invention, showing a needle seat being wedged into a syringe.

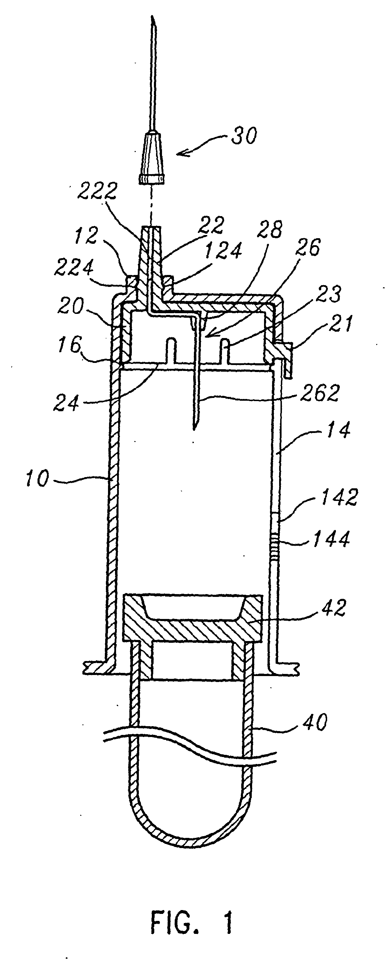

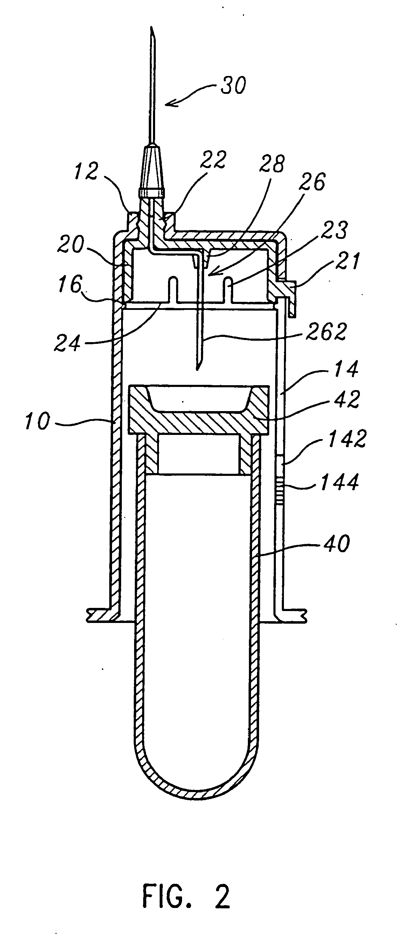

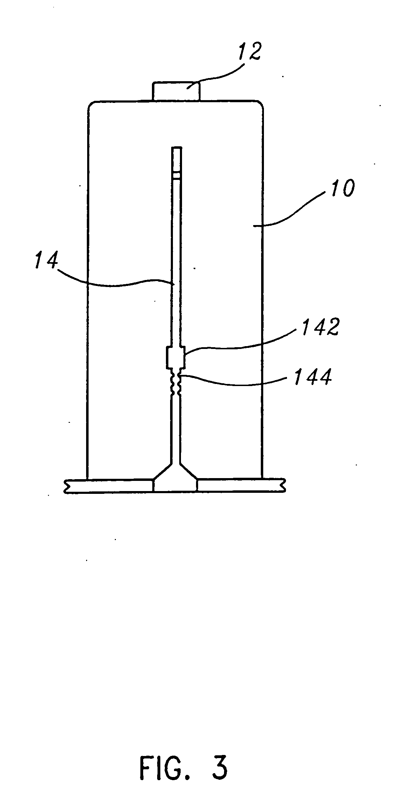

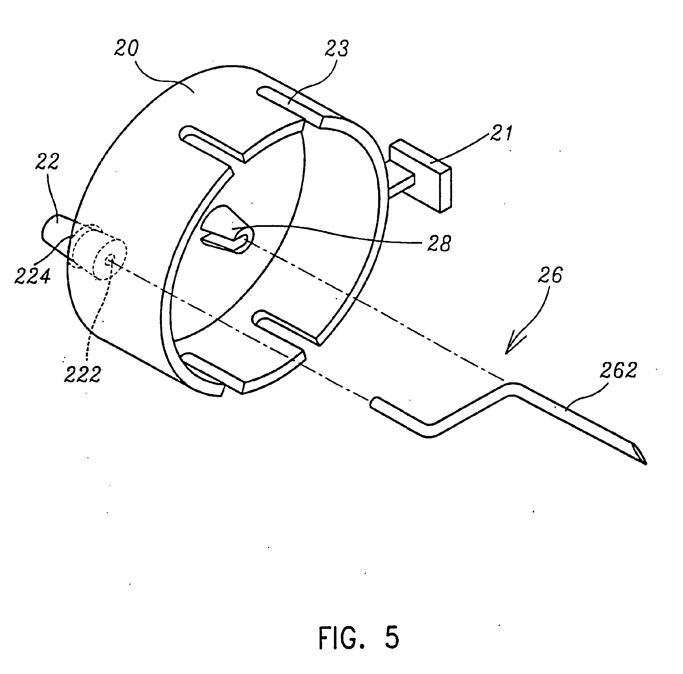

As shown in FIG. 1, 2 and 3 an example comprises a hollow barrel, a reduced inlet 12 eccentrically disposed at the top end of the barrel 10 and a guide slit 14 at the flank side of the barrel 10. The barrel comprises a reduced lining tube 20, an eccentric reduced portion 22 is disposed at a position away from the center of the top end of the reduced lining tube 20 and an opening 24 is disposed at the bottom thereof. The eccentric reduced portion 22 can be embedded into the reduced inlet 12 and projects out of the reduced inlet 12. A needle head 30 can be wedged onto the eccentric reduced portion 22, and a through guiding hole 222 is disposed at the center of the eccentric reduced portion 22 so that a step shaped needle 26 can be fixed therein and a lower needle tip 262 of the needle 26 can be placed at the central axial line of the reduced lining tube 20. A fixing seat 28 is disposed at the center of the inner section of the reduced lining tube 20 for receiving and fixing the needle 26 therein. As FIG. 5 shown, a press plate 21 is further attached to the reduced lining tube 20 at the outer surface thereof and positioned in the guiding slit 14 to be utilized to drive the reduced lining tube to move in the longitudinal direction of the barrel 10.
A hook-shaped flange 16 is further disposed at a proper position of the inner surface of the barrel 10 so as to position the reduced lining tube 20 at the top end of the barrel 10 and not to slide loosely therein. Furthermore, at least one circular flange 224 is disposed around the eccentric reduced portion 22 and at least one circular groove 124 is disposed around the inner surface of the reduced inlet 12 to accommodate and position the circular groove 124. A plurality of small slits 23 are cut around lower side of the reduced lining tube 20, enabling the lower end of the reduced lining tube to maintain its proper elasticity. A vacuum tube 40 is further disposed inside the barrel 10, and an elastic plug 42 is fixedly covered in an opening that is at the top end of the vacuum tube 40: The vacuum tube 40 is commercially available.

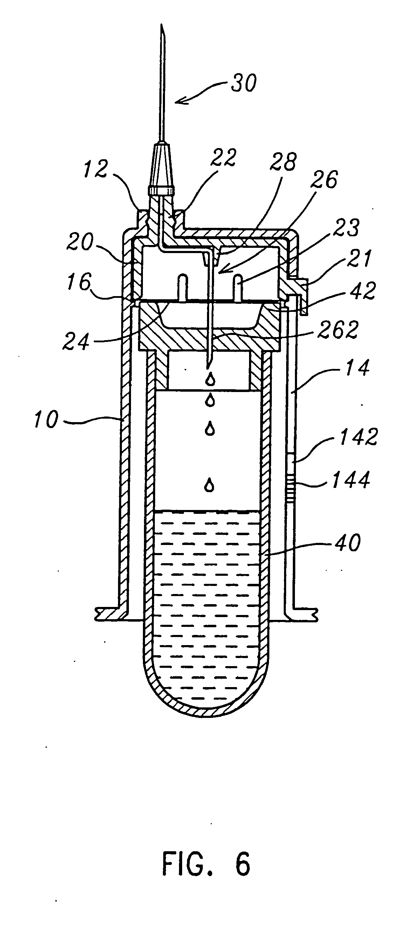

Please refer to FIG. 6, when processing a blood sampling, strike the needle head 30 is inserted into the vein of a patient, and the vacuum tube 40 moves toward the needle 26 inside the barrel 10 in order to make the lower needle tip 262 to prick through the plug 42 on the top of the cup 40. The blood from the vein of the patient will pass through the needle head 30, the guiding hole 222, the needle 26, and into the vacuum blood collecting cup 40. Since the needle head 30, the eccentric reduced portion 22 and reduced inlet 12 are all eccentrically disposed and close to the circumference of the barrel 10, a health care worker can prick the needle head 30 into the vein with a smaller inclined angle to the skin of the patient. Thus, the vacuum syringe not only conforms to ergonomics, but prevent from the needle head piercing through the blood vessels.

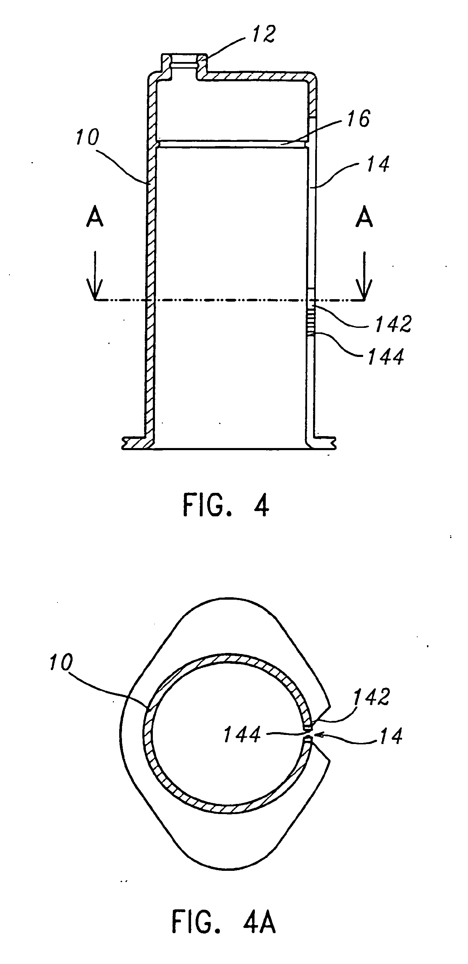

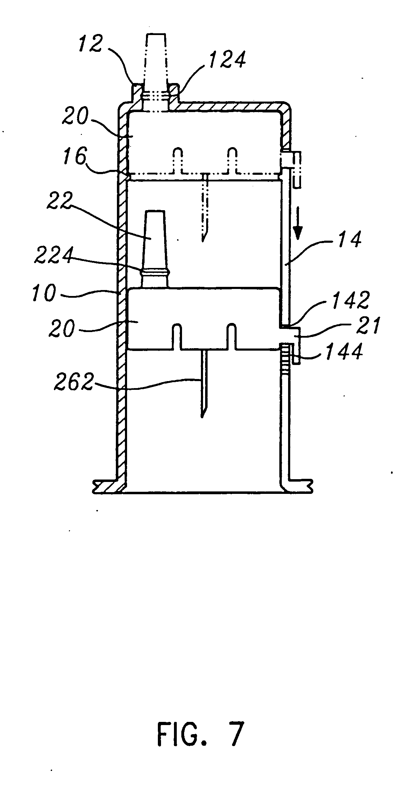

Refering to FIG. 7, after the blood sampling is over, the press plate is pressed 21 backward to move the reduced lining tube back to prevent the needle head from pricking the health care workers. A stopping groove 142 is disposed at a proper position of the guiding slit 14, the width of the stopping groove 142 is larger than the width of the guiding slit 14 in order to stop the press plate 21 to moving forward or backward when the press plate 21 is moved to the stopping groove 142 to avoid the lower needle tip 262 at the lower end of the needle 26 to project out of the barrel 10 to cause danger. A plurality of bulging points 144 are disposed at the section of the guiding slit 14 below the stopping groove 142 to prevent the press plate 21 from slipping backward to drop out of the stopping groove 142 resulting that the lower needle tip being exposed from the barrel 10, as shown in FIG. 4 and 4A.

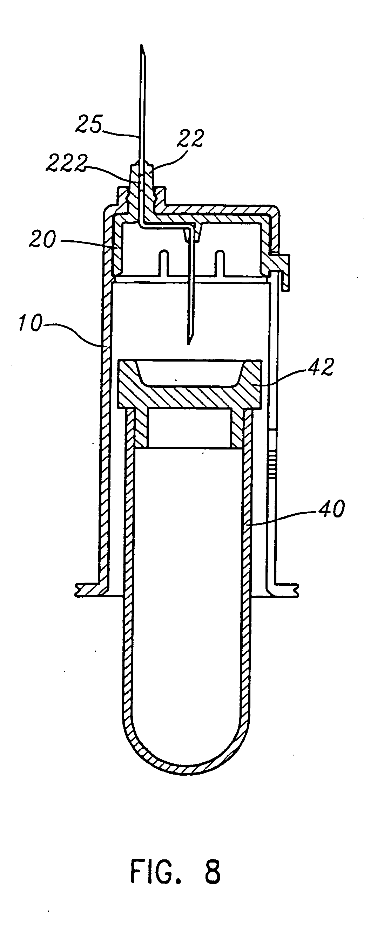

Referring also to FIG. 8, a cannula needle can be located in the guiding hole 222 in advance when the eccentric reduced portion 22 of the reduced lining tube 20 is in production. Thus, there is no need to insert the needle separately, saving time and production cost.

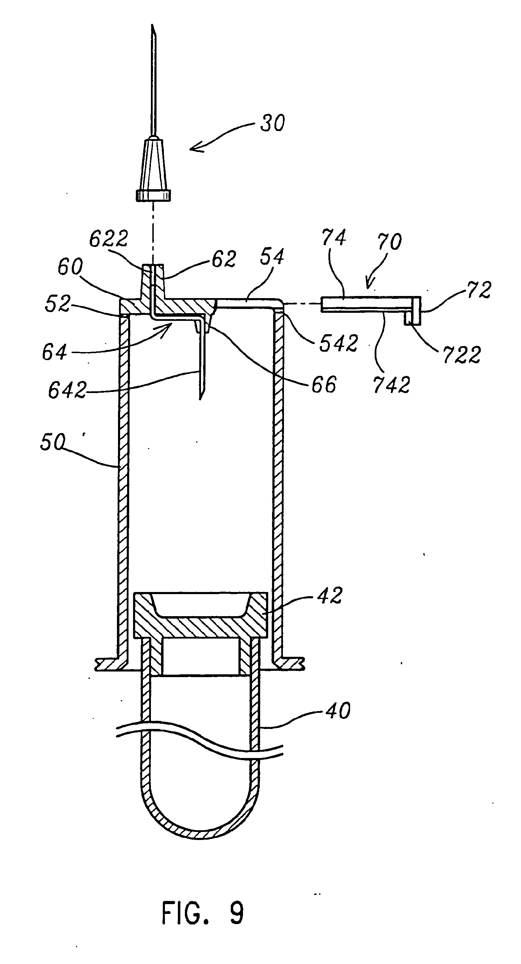

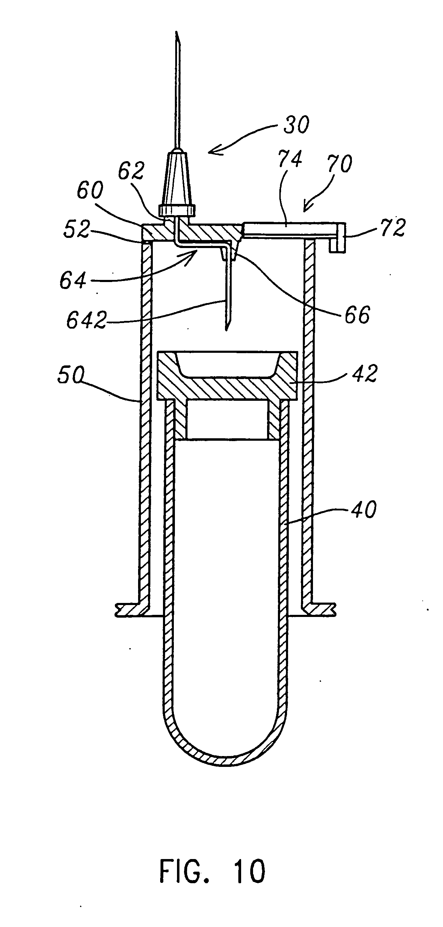

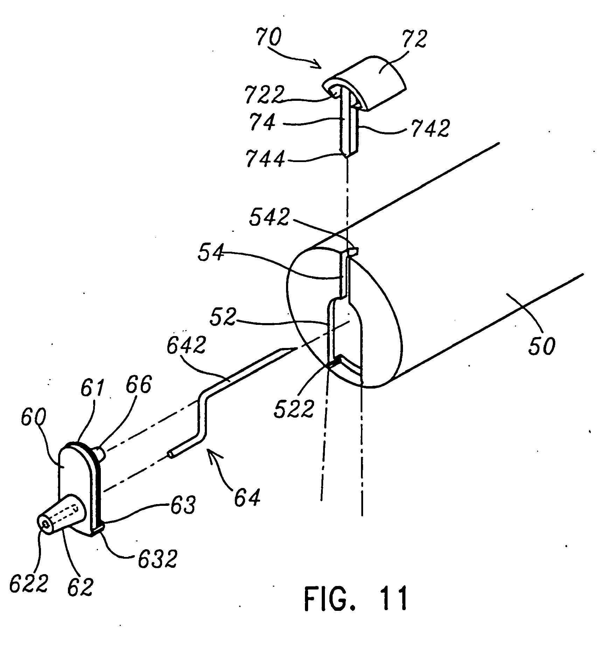

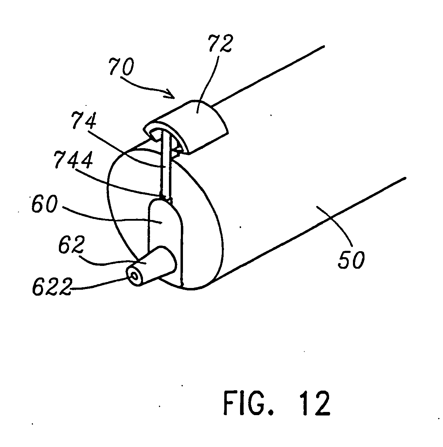

FIGS 9, 10, 11 and 12 show preferred embodiment of the present invention comprising a hollow barrel 50, a directional sliding trench 52 is transversely disposed at one side of the front end of the barrel 50, and a sliding slit 54 transversely disposed at another side opposite to the trench. A needle seat 60 and a press plate 70 are further disposed in the trench 52 and the slit 54 respectively. An eccentric reduced portion 62 is still disposed on the upper surface of the needle seat, and a needle head 30 may be wedged on the eccentric reduced portion 62. A guiding hole is pierced through the eccentric reduced portion 62 so that a step shaped needle comprising two right angles can be fixed therein and a lower needle tip 642 of the needle 64 can be placed at the central axial line of the reduced lining tube 50 firmly. A fixing seat 66 is disposed at the lower side of the needle seat 20 for receiving and fixing the needle 64 therein. Because the needle seat 60 can be fixed firmly in the trench 52, a positioning flange 61 is disposed around the outside profile of the needle seat 60, and a small flange 632 is attached to each flank side of a convex plate 63. A guiding groove 522 is disposed at each flank side of the trench corresponding to the position of the flange 632 so as to fix the flange therein to prevent the needle seat from dropping out of the trench. Moreover, in order to assemble the needle seat 60 easily into the trench 52, the trench 52 has a certain degree of angle between its two sides so that the width of the opening at the front edge is larger than the one of the end, enabling the needle seat 60 to easily slip into the trench 52. The press plate 70 has an end face 72 and a guiding stick 74, the guiding stick 74 has a triangle shape strip 742 at its flank side. The sliding slit 54 has a triangle shape slit opening 542 at its flank side corresponding to the position of the strip 742, so as to move the press plate 70 as a guide. A triangle shape inverted hook is formed at the end of the guiding stick 74 in order to be connected with the joint of the slit 54 and the trench 52 when the press plate 70 is positioned in the slit 54, enabling the press plate 70 not to slide backward to separate from the slit 54. An arc spring plate 722 is connected to the end of each flank side below the end face 72 to provide proper elastic force. A vacuum tube 40 is further disposed in the barrel 50, and an elastic plug 42 is firmly covered in the opening at the upper end of the vacuum tube 40, wherein the vacuum tube is available in the market.

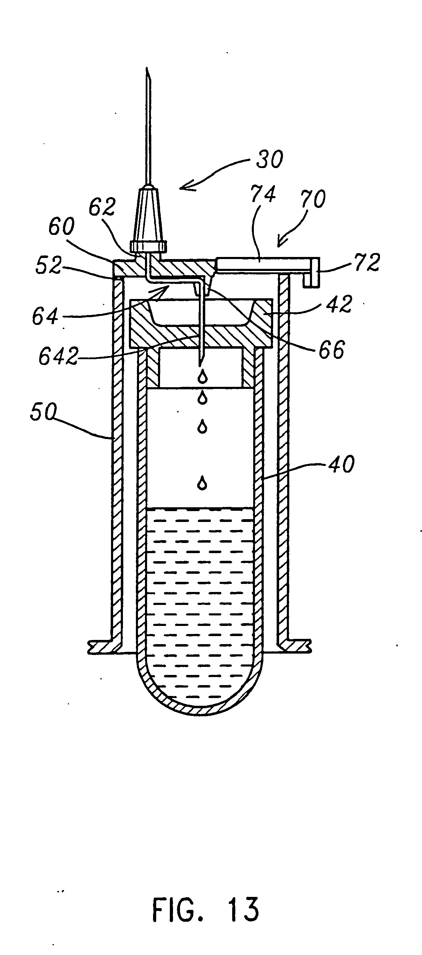

Next, referring to FIG. 13, when taking sample a blood, strike the needle head into the vein of a patient, then move the vacuum tube 40 toward the needle 64 inside the barrel 10 to allow the plug 42 of the cup 40 to be pierced. The blood from the vein of the patient will pass through the needle head 30, the guiding hole 622, to the needle 64, and into the vacuum blood collecting cup 40. When the blood sampling is over, the press plate 70 may be pushed to separate the needle seat 60 from the trench, and then to throw it in a needle head collector. Therefore, change the needle seat 60 and needle head for the next blood sampling to avoid the infection of virus.

The advantage of the invention is that a proper thickness needle head 30 may be chosen to wedge on the eccentric reduced portion 22, 62, and the needle head can be a conventional needle head instead of using a particular one that needs a new mold to produce saving costs. Moreover, since a conventional vacuum blood collecting cup can still be used after the needle head 30 is eccentrically disposed, the needle 26, 64 of the invention is designed to be like a step comprising two right angles shape so as to position the lower needle tip 262, 642 of the needle 26, 64 at the center inside the barrel 10 and 50. Therefore, the lower needle tip 262, 642 can prick through the plug at the center, alleviating the need for vacuum blood collecting cup is unnecessary.

It is to be understood that the drawing is designed for purpose of illustration only, and is not intended for use as a definition of the limits and scope of the invention disclosed.

## Claims

1. A safety vacuum syringe for blood sampling conformed to ergonomics, mainly comprising:
a hollow barrel (50),
a directional sliding trench (52) disposed transversely at one side of the front end of said barrel, a needle seat (60) disposed in said trench,
an eccentric reduced portion (62) disposed on the upper surface of the needle seat,
a guiding hole disposed through the eccentric reduced portion to fix a step shaped needle comprising two right angles therein and a lower needle tip (642) of said step shaped needle placed at the central axial line of the reduced lining tube,
a needle head covered on said eccentric reduced portion,
a vacuum tube, disposed in said barrel, and an elastic plug covered on an opening at the upper side of said vacuum tube,
**characterized in that** it further comprises a sliding slit (54) disposed transversally at another side of the front end of the barrel opposite to said trench and a press plate (70) disposed in said slit (54), the press plate (70) having an end face (72) and a guiding stick (74), said guiding stick having a triangle shape strip (742) at its flank side, said sliding slit having triangle shape slit opening at its flank side corresponding to the position of the strip, so as to move the press plate as a guide.

2. The syringe of claim 1, wherein a positioning flange (61) is disposed around the outside profile of said needle seat, and a small flange (632) is attached to each flank side of a convex plate, a guiding groove is disposed at each flank side of the trench corresponding to the position of said small flange so as to fix the flange therein to prevent the needle seat from dropping out of the trench.

3. The syringe of claim 1, wherein a fixing seat (66) is disposed at the lower side of said needle seat for receiving and fixing the needle therein.

4. The syringe of claim 1, wherein a triangle shape inverted hook is formed at the end of said guiding stick in order to connect with the joint of said slit and said trench when said press plate is positioned in said slit.

5. The syringe of claim 1, wherein an arc spring plate is connected to the end of each flank side below the end face to provide proper elastic force.

6. The syringe of claim 1, wherein said trench has a certain degree of angle between its two sides so that the width of the opening at the front edge is larger than the one of the end.

## Patentansprüche

1. Ergonomisch gestaltete Sicherheitsvakuumspritze zur Blutentnahme, die hauptsächlich Folgendes umfasst:
einen hohlen Zylinder (50),
einen gerichteten Schiebekanal (52), der quer auf einer Seite des vorderen Endes des Zylinders angeordnet ist, wobei in dem Kanal eine Nadelaufnahme (60) angeordnet ist,
einen exzentrischen verjüngten Abschnitt (62), der auf der Oberseite der Nadelaufnahme angeordnet ist,
ein Führungsloch, das in dem exzentrischen verjüngten Abschnitt angeordnet ist, um eine stufenförmige Nadel mit zwei rechten Winkeln darin zu befestigen, wobei eine untere Nadelspitze (642) der stufenförmigen Nadel auf der axialen Mittellinie des verjüngten Auskleidungsröhrchens liegt,
einen Nadelkopf, der auf dem exzentrischen verjüngten Abschnitt angeordnet ist, ein Vakuumröhrchen, das in dem Zylinder angeordnet ist, und einen elastischen Stopfen, der auf einer Öffnung auf der Oberseite des Vakuumröhrchens angeordnet ist,
**dadurch gekennzeichnet, dass** sie ferner einen Schiebeschlitz (54) umfasst, der quer auf der anderen Seite des vorderen Endes des Zylinders gegenüber dem Kanal angeordnet ist, und eine in dem Schlitz (54) angeordnete Pressplatte (70), wobei die Pressplatte (70) eine Stirnfläche (72) und einen Führungsstift (74) aufweist, wobei der Führungsstift auf seiner Flankenseite einen dreieckförmigen Streifen (742) aufweist, wobei der Schiebeschlitz auf seiner Flankenseite eine dreieckförmige Schlitzöffnung aufweist, die der Position des Streifens entspricht, um die Pressplatte als Führung zu bewegen.

2. Spritze nach Anspruch 1, wobei ein Positionierungsflansch (61) um das äußere Profil der Nadelaufnahme herum angeordnet ist, ein kleiner Flansch (632) an jeder Flankenseite einer konvexen Platte befestigt ist und eine Führungsnut auf jeder Flankenseite des Kanals entsprechend der Position des kleinen Flansches angeordnet ist, um den Flansch darin zu befestigen, um zu verhindern, dass die Nadelaufnahme aus dem Kanal herausfällt.

3. Spritze nach Anspruch 1, wobei eine Befestigungsaufnahme (66) auf der Unterseite der Nadelaufnahme angeordnet ist, um die Nadel darin aufzunehmen und zu befestigen:

4. Spritze nach Anspruch 1, wobei ein dreieckförmiger umgedrehter Haken am Ende des Führungsstifts ausgebildet ist, um mit dem Gelenk des Schlitzes und dem Kanal verbunden zu werden, wenn die Pressplatte in dem Schlitz positioniert ist.

5. Spritze nach Anspruch 1, wobei eine Federplatte mit dem Ende jeder Flankenseite unter der Stirnfläche verbunden ist, um die entsprechende Federkraft bereitzustellen.

6. Spritze nach Anspruch 1, wobei der Kanal ein gewisses Maß an Winkel zwischen seinen beiden Seiten aufweist, so dass die Breite der Öffnung am vorderen Rand größer ist als die am Ende.

## Revendications

1. Seringue à aspiration de sécurité et ergonomique pour prise de sang, comprenant principalement :
un cylindre creux (50),
une rainure de glissement directionnelle (52) disposée transversalement sur un côté de l'extrémité avant dudit cylindre, un siège d'aiguille (60) étant placé dans ladite rainure,
une partie réduite excentrée (62) disposée sur la surface supérieure du siège d'aiguille,
un trou de guidage traversant la partie réduite excentrée pour fixer une aiguille coudée comprenant deux angles droits et un bout d'aiguille inférieur (642) de ladite aiguille coudée placé sur l'axe du tube de doublage réduit,
une tête d'aiguille couverte sur ladite partie réduite excentrée,
un tube à vide, disposé dans ledit cylindre, et un bouchon élastique couvert sur une ouverture prévue dans le côté supérieur dudit tube à vide,
**caractérisé en ce qu'**il comprend en outre une fente de glissement (54) disposée transversalement sur un autre côté de l'extrémité avant du cylindre, en face de ladite rainure, et une plaquette d'actionnement (70) placée dans ladite fente (54), la plaquette d'actionnement (70) ayant une face d'extrémité (72) et un axe de guidage (74), ledit axe de guidage comportant une bande de forme triangulaire (742) sur son côté latéral, ladite fente de glissement ayant une ouverture de fente de forme triangulaire sur son côté latéral correspondant à la position de la bande, afin de déplacer la plaquette d'actionnement comme un guide.

2. Seringue selon la revendication 1, dans laquelle une bride de positionnement (61) est disposée autour du profil extérieur dudit siège d'aiguille, et une petite bride (632) est solidaire de chaque côté latéral d'une plaquette convexe, une rainure de guidage est disposée dans chaque côté latéral de la rainure en correspondance avec la position de ladite petite bride afin d'y fixer la bride pour empêcher le siège d'aiguille de sortir de la rainure.

3. Seringue selon la revendication 1, dans laquelle un siège de fixation (66) est placé dans la partie inférieure dudit siège d'aiguille pour y recevoir et y fixer l'aiguille.

4. Seringue selon la revendication 1, dans laquelle un crochet inversé de forme triangulaire est formé à l'extrémité dudit axe de guidage de façon à se connecter à la jonction de ladite fente et de ladite rainure lorsque ladite plaquette d'actionnement est positionnée dans ladite fente.

5. Seringue selon la revendication 1, dans laquelle une plaquette ressort arquée est connectée à l'extrémité de chaque côté latéral sous la face d'extrémité pour fournir une force élastique appropriée.

6. Seringue selon la revendication 1, dans laquelle ladite rainure présente un certain angle entre ses deux côtés, de sorte que la largeur de l'ouverture au niveau du bord avant est plus grande que celle de l'extrémité.
